(19) Europäisches Patentamt
European Patent Office
Office européen des brevets

(11) Numéro de publication: **0 118 376**
**B1**

(12) **FASCICULE DE BREVET EUROPÉEN**

(45) Date de publication du fascicule du brevet: **02.09.87**

(51) Int. Cl.⁴: **C 12 P 19/18,** C 12 N 1/00

(21) Numéro de dépôt: **84420028.7**

(22) Date de dépôt: **22.02.84**

(54) **Substance gélifiante semi-synthétique, son procédé de préparation et ses applications, notamment dans les milieux de culture pour micro-organismes.**

(30) Priorité: **23.02.83 FR 8303368**

(43) Date de publication de la demande:
**12.09.84 Bulletin 84/37**

(45) Mention de la délivrance du brevet:
**02.09.87 Bulletin 87/36**

(84) Etats contractants désignés:
**AT BE CH DE FR GB IT LI LU NL SE**

(56) Documents cité:
**GB-A-976 224**
**GB-A-2 082 189**

**CHEMICAL ABSTRACTS, vol. 95, 1982, page 102, no. 205781p, Columbus, Ohio, US**
**JOURNAL OF CHROMATOGRAPHY, vol. 128, 1976, pages 125-131, Elsevier Scientific Publishing Company, Amsterdam, NL, H. PERTOFT et al.: "Preparation of silica-agarose beads for gel chromatography"**

(73) Titulaire: **SOCIETE A RESPONSABILITE LIMITEE DITE "KORANO", La Balme Les Grottesers, F-38390 Montalieu Vercieu (FR)**

(72) Inventeur: **Brochon, Marie- José, 8 Bis rue Dr F. Arnaud, Vanves Hauts de Seine (FR)**
Inventeur: **Corot, Anne- France, 312 rue des Marronniers, Olivet Loiret (FR)**

(74) Mandataire: **Maureau, Pierre, Cabinet GERMAIN & MAUREAU Le Britannia - Tour C 20, Boulevard E. Déruelle, F-69003 Lyon (FR)**

**EP 0 118 376 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne une substance gélifiante semi-synthétique, son procédé de préparation et ses applications, notamment comme substitut de l'agar-agar dans les milieux de culture pour microorganismes.

Les opérations d'isolement de microorganismes couramment réalisées dans les laboratoires d'analyse microbiologique imposent d'effectuer un ensemencement de ces microorganismes dans ou sur un milieu de culture ou ils se développent dans des conditions physicochimiques bien déterminées. Cet ensemencement se fait fréquemment à la surface d'un milieu de culture solide constitué d'éléments nutritifs contenus dans un gel. Chaque bactérie en se développant forme une colonie isolée.

On connaît de nombreuses substances susceptibles de former, en milieu aqueux, des gels susceptibles de se solidifier; c'est le cas, par exemple, de produits macromoléculaires synthétiques (polyacrylamide, polycarboxyvinyle...) ou de substances minérales comme les silicates. Mais ces substances ne sont pas adaptées à la formation ni à la préparation facile de milieux de culture car ils ne sont pas réversibles; de plus, dans le cas de macromolécules de synthese, la necessité de leur adjoindre un agent de polymérisation pour provoquer la formation de gel les rend incompatibles avec la préparation de milieux de culture à partir de poudres sèches par simple addition d'eau.

Il existe également de nombreuses macromolécules naturelles susceptibles de former des gels sous l'action de la température; c'est le cas, par exemple, de la gélatine et des pectines, qui sont compatibles avec les milieux de culture mais ont des applications limitées en bactériologie; en effet, ces gels sont attaqués par de nombreuses bactéries.

Jusqu'à ce jour, seul l'agar-agar naturel possède les caractéristiques permettant son utilisation universelle en bactériologie. Il est le seul produit a donner des gels réversibles et stables dont la température de fusion (100° C) soit très supérieure à la temperature de gélification (40° C). Il s'agit, de plus, d'une substance qui n'est pas attaquée rapidement par les bactéries et qui ne présente aucune toxicité.

L'agar-agar naturel ou gélose est obtenu par extraction à partir de différentes algues marines appartenant é la classe des Rhodophyceae. Il s'agit d'un mélange complexe de polysaccharides presentant tous le squelette d'un polygalactane formé par la répetition alternative d'unites de (1-3)(βD-galactose et de (1-4)3,6-anhydro-αL-galactose, désigné sous le nom d'agarose et qui, sous sa forme commerciale, est toujours plus ou moins substitué; l'agarose pure n'existe pas en effet dans la nature.

Il s'agit d'un produit pratiquement insoluble dans l'eau à 25°C, qui se disperse dans l'eau vers 100°C en donnant des solutions colloïdales dont la viscosité varie en fonction de l'origine et de la concentration; à une concentration d'au moins 0,5 % il forme, par refroidissement, des gels qui ont une bonne stabilite et sont réversibles (ils fondent à chaud et se regélifient a temperature inferieure à 40°C). Ces gels sont caractérisés par leur température de gélification dynamique (passage de l'état de solution à l'état de gel par refroidissement) et isothermique (température minimale a laquelle une solution d'agar-agar peut être maintenue indéfiniment sans former de gel). Ces paramètres varient également en fonction de l'origine de l'agar-agar, de son poids moleculaire, des lots de fabrication et de la concentration du gel.

La préparation de milieux de culture solides utilisant l'agar-agar comme produit gélifiant consiste à mélanger cet agar-agar à des substances nutritives convenables, puis, après addition d'eau, à solubiliser l'ensemble tout en le rendant stérile par passage a l'autoclave.

Il existe actuellement des poudres sèches, prêtes à l'emploi, renfermant, en mélange, l'agar-agar et les substances nutritives convenables; ces poudres, non stériles, sont commercialisées par lots d'au moins 500 grammes. La préparation d'un milieu de culture à partir de ces poudres impose la suite d'opérations suivantes:

- pesage de la quantité désirée (p.ex. 40 g pour 1 litre d'eau)
- dissolution, à l'ébullition, de cette poudre dans l'eau
- passage de cette solution a l'autoclave à 120° C pendant au moins 20 minutes
- répartition dans les récipients de culture (boîtes de Pétri...) manuellement ou par l'intermédiaire d'une machine.

L'ensemble de ces opérations nécessite au moins une heure, ce qui exclut le conditionnement de ces poudres en dose unitaire (quantité de poudre nécessaire pour faire un seul test de culture). On prépare toujours au moins 20 à 40 boîtes de Pétri à la fois, ce qui présente d'autres inconvenients en raison notamment des variations quotidiennes de consommation d'un laboratoire qu'il est souvent difficile de prévoir.

La présente invention vise a pallier ces inconvénients.

Les inventeurs ont réussi à mettre au point une nouvelle substance gélifiante semi-synthétique susceptible de remplacer l'agar-agar et dont l'emploi, notamment dans les milieux de culture, sous forme de poudres sèches, permet de réduire de façon importante la durée de préparation de ces milieux en supprimant notamment la nécessité de passage en autoclave pour parfaire sa dissolution et assurer sa stérilité; il est ainsi possible de préparer ces milieux de culture sous forme de doses unitaires en conditionnant, dans un même récipient stérile et manipulable stérilement, la substance gélifiante et la quantité dosée de poudre stérile de substances nutritives.

C'est ainsi que l'invention a pour objet une nouvelle substance gélifiante dont l'originalité consiste en ce qu'elle est essentiellement constituée de chaines enchevêtrées et relativement courtes de polygalactanes non ramifiées, où alternent les forme D et L, comme dans l'agar-agar, ce qui constitue l'originalité essentielle de l'invention car il n'existe aucune autre substance obtenue par extraction ou par synthese chimique possédant

ce squelette. Cette substance différe toutefois de l'agar-agar parce que ces chaînes sont plus courtes, enchevétrées et réunissent des grains d'oxydes métalliques inertes tels que les oxydes d'aluminium, de silicium et de titane. La proportion d'oxydes métalliques par rapport aux polygalactanes est inversement proportionnelle à la longueur des chaînes polygalactanes et se situe avantageusement entre 10 et 80 %.

La substance gélifiante selon l'invention, que l'on désignera dans ce qui suit sous l'appellation de polygalactane de synthèse-oxyde ou PGS-O, présente des propriétés physico-chimiques voisines de celles de l'agar-agar: il est pratiquement insoluble dans l'eau à 25°C mais se disperse dans l'eau à partir de 80°C; il forme un gel par refroidissement; en solution dans l'eau, il donne des dispersions dont la viscosité varie avec la concentration; à concentration d'au moins 0,5 %, il forme des gels stables et réversibles.

Le PGS-O présente toutefois, par rapport à l'agarose, les différences essentielles et caractéristiques suivantes:

- Dès 80°C, le PGS-O forme des solutions, alors qu'à la même température les grains d'agar-agar, même pulvérulents, gonflent sans se dissoudre. La caractéristique essentielle du PGS-O est donc de se dissoudre complètement dans l'eau dès 80°C.

- Les chaînes d'agarose ne forment de ponts qu'entre elles, ce qui conduit à l'obtention de gels d'aspect cristallisé; par contre, les chaînes de PGS-O réunissent des grains d'oxydes métalliques et gélifient donc une suspension de grains d'oxydes dans l'eau. La figure 1 du dessin schématique annexé représente cette structure.

- Les chaînes de PGS-O sont beaucoup plus petites que les chaînes d'agarose. La longueur des chaines est mesurée par les poids moléculaires puisqu'il s'agit de la répétition du même disaccharide non substitué, de poids moléculaire 325 ($H_2O$). Les chaînes utilisables de PGS-O ont un poids moléculaire qui varie entre 6000 et 15000, le nombre de disaccharides n varie donc de 19 à 40-50. Dans l'agarose n est très variable selon les origines des agars naturels (entre 20 et 500). Le poids moléculaire du PGS-O est mesuré pour chaque lot de fabrication et il est indiqué à l'utilisateur (par ex.: produit contenant un PGS-O 6000, 8000...) ce qui n'est pas possible avec l'agar-agar; cette détermination demanderait d'effectuer une vérification sur tamis moléculaire, l'agar-agar étant toujours constitué d'un mélange de polysaccharides ayant un même squelette plus ou moins substitué.

- Les poudres de PGS-O sont blanches et sous forme de fines paillettes de moins de un centième de millimètre d'épaisseur. Pour les PGS-O utilisés en bactériologie leur densité apparente est plus faible que celle de l'agar-agar. Par exemple un PGS-O de qualité bactériologique, de PM 12000 et contenant $Al_2O_3$, $TiO_2$ et $SiO_2$ dans les proportions 66.17.16.- 01 (voir exemple 1) a une densité apparente de 0,118 alors que l'agar-agar bactériologique, utilisé pour les mêmes applications, a une densité apparente de 0,384 et forme des grains. (La densité apparente d'une poudre est une mesure utilisée en pharmacie galénique. Elle définit le poids d'un certain volume de poudre non tassée: par exemple, un litre de PGS-O 66.17.16.01 non tassé pèse 118 g.)

En revanche, la densité réelle est plus grande avec le PGS-O qu'avec l'agar-agar (voisine de 1 pour l'agar-agar et de 1,3 pour un PGS-O 66. 17.16.01, en raison de la présence d'oxydes métalliques).

- Les poudres de PGS-O sont compatibles avec la granulation et la compression: les particules de PGS-O sont capables de s'agglutiner et de rester liées les unes aux autres après la compression. On obtient des granulés ou des comprimés solides, non friables et qui s'élident dans l'eau à 80-100°C, ce qui est impossible avec l'agar-agar du commerce.

- Dans les gels d'agarose, les chaînes sont parallèles et ordonnées, alors que dans les gels de PGS-O elles sont enchevêtrées comme les fibres de cellulose du papier. Cette caractéristique est facile à vérifier par diffraction aux rayons X: l'agarose montre une structure répétitive (domaine cristallin) tandis que le PGS-0 ne laisse apparaître que des halos de diffraction et ne présente pas de régularité de structure (domaine amorphe).

- L'analyse chimique permet, bien entendu, de différencier l'agarose (qui ne contient que des polygalactanes) des différents PGS-O selon l'invention, qui sont caractérisés par leur teneur en différents oxydes métalliques.

- Les spectres en RMN de l'agarose et du PGS-O, tels qu'ils sont représentés en figures 3 et 4 du dessin schématique annexé, tout en étant voisins, sont très reconnaissables.

Les spectres obtenus par RMN au carbone 13 à partir d'agar-agar (figure 3) du commerce permettent de mettre en évidence 12 signaux différents principaux correspondant aux atomes de carbone, et indiquent que l'agarose possède une structure régulière où se répète une même unité de disaccharide. Les signaux enregistrés à 103,4 et 99,2 p.p.m. sont attribués aux carbones $C_1$ du β-D-galactopyranosyl et $C_1$ du 3,6 anhydro-α-L-galactopyranosyl; le signal à 62,4 p.p.m. est attribué au carbone en $C_6$ du D-galactose. Les autres signaux correspondent soit aux 9 autres carbones constituant l'unité de disaccharide, la hauteur des pics variant avec la nature des substituants sur ces carbones, soit à des substituants comme les groupes méthoxyl à 60 p.p.m.

Avec les PGS-O (figure 4), les oxydes donnent des absorptions caractéristiques que l'on ne retrouve pas dans l'agarose. Dans la zone correspondant aux polygalactanes, on retrouve les 12 pics principaux correspondant a chacun des carbones, mais les PGS-O ne donnent pas de pics correspondant à des substituants, et la hauteur des pics obtenus est fixe (contrairement à l'agar-agar du commerce). Par contre on retrouve, comme pour l'agar-agar, les pics à 103,4 et 99,2 caractéristiques de l'alternance des formes D et L galactose.

Les spectres RMN au carbone 13 apparaissent donc comme le meilleur procédé de contrôle de fabrication des PGS-O.

- Enfin, à poids de matière séche égal, le gel de PGS-O est blanc, plus ou moins opaque, tandis que le gel

d'agarose est plus ou moins transparent.

Il convient, par ailleurs, de noter que la simple addition, à l'agarose, de proportions d'oxydes métalliques similaires à celles que renferme le PGS-O ne modifie pas les propriétés de l'agarose et ne lui communique nullement celles du PGS-O, en particulier de donner des solutions vers 80° C.

L'invention a également pour objet un procédé de fabrication bio-synthétique du polygalactane de synthèse-oxyde, selon lequel on utilise des microorganismes producteurs d'enzymes pour synthétiser, à partir d'oligosaccharides provenant de matières premières contenant déjà des D et L galactoses, un squelette de polygalactane non ramifié où alternent les formes D et L, la culture desdits microorganismes étant effectuée a travers une membrane poreuse sur laquelle sont étalées des poudres fines d'oxydes métalliques.

Selon un mode de réalisation préféré de l'invention, les microorganismes sont tout d'abord isolés à partir d'oligo-saccharides provenant de l'hydrolyse de l'agarose. On sait que certains microorganismes possèdent des enzymes qui hydrolysent l'agarose et libèrent des oligo-saccharides:

- 4-O-β-D-galactopyranosyl(1→4)3,6 anhydrod-α-L-galactose, correspondant a la répétition d'unités d'AGAROBIOSE (oligosaccharides A-B).

- O-3,6 anhydro-α-L-galactopyranosyl(1→3)β-D-galactose correspondant à la répétitions d'unités de NEOAGAROBIOSE (oligosaccharides B-A).

Ces enzymes coupent les liaisons β-D-(1→4) et α-L-(1→3)-galactose, selon le schéma suivant:

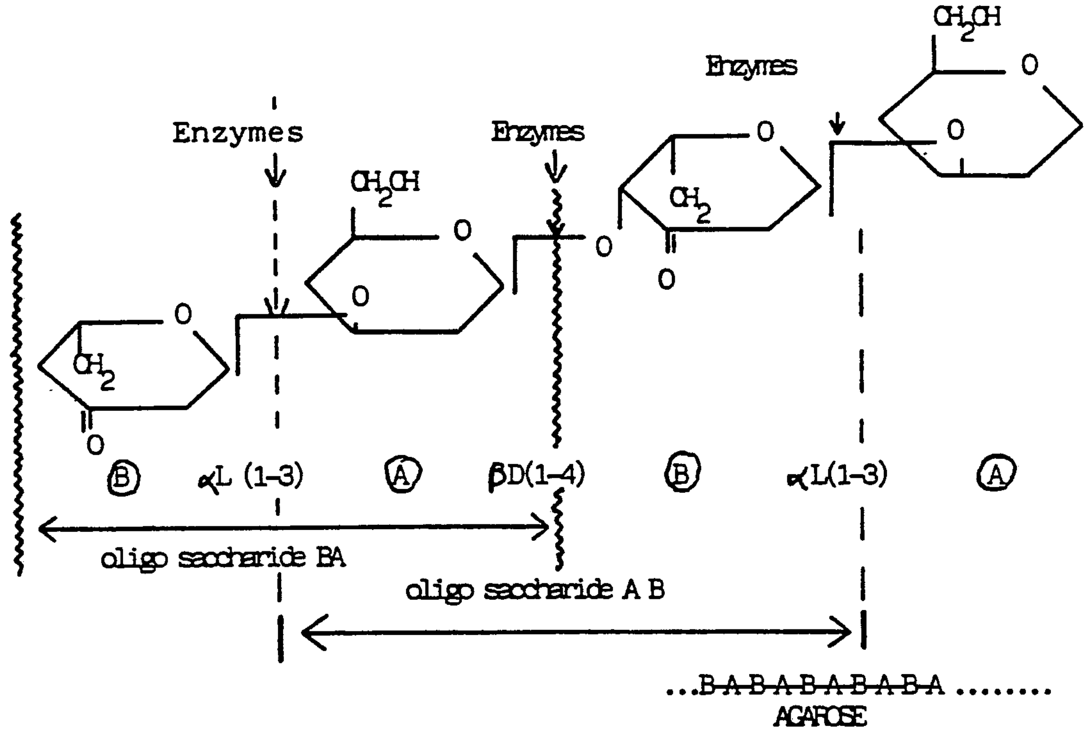


Il est à noter que les enzymes ont une action réversible et que le même microorganisme qui possède la propriété d'hydrolyser peut posséder inversement la propriété de polymériser.

Ces oligosaccharides, préparés à partir de l'agarose, serviront à sélectionner les microorganismes producteurs de polymérases.

L'isolement des microorganismes capables de synthetiser l'agarose ou le polygalactane de synthèse à partir d'oligosaccharides se fait selon le processus suivant:

- Avec les oligosaccharides provenant de l'hydrolyse de l'agarose, on prépare stérilement un milieu de culture solide synthétique. A la surface de ce milieu, on isole des variétés myxoïdes de bactéries connues pour produire des polysaccharides capsulaires. Ces bactéries sont nombreuses (Pseudomonas, Agrobactérium, Entérobactéries...). On peut donc utiliser avec profit une bactérie bien connue pour hydrolyser l'agarose Pseudomonas Atlantis et sélectionner des variétés myxoïdes (qui ont des aspects muqueux dus aux polysaccharides)

-Par screening et par clonage, on recherche les souches capables de synthétiser un polysaccharide dont la structure est voisine de celle de l'agarose c'est-à-dire que l'on recherche des souches qui sont capables, à partir des oligosaccharides du milieu, de faire des chaînes plus longues. Ces oligosaccharides, qui contiennent 2, 3 ou plus unités de disaccharides sont utilisés comme initiateurs. Les microorganismes capables d'allonger les chaînes le font à l'aide d'hexose transferase (enzyme qui allonge les chaînes en ajoutant hexose après hexose). Des hexoses transférases spécifiques des liaisons (1→3) et (1→4) allongent les chaînes selon des procédés de polymérisation connus en biologie. L'alternance des formes D et L galactose et des liaisons (1→3) et (1→4) est obtenue en faisant agir sur les oligosaccharides A-B une (1→3) β-D-galactose transférase et sur les oligosaccharides B-A une (1→4) à α-L-galactose transférase.

Bien que ce screening soit relativement long, même en partant de bactéries marines, il est relativement facilité parce qu'on recherche un polysaccharide insoluble dans l'eau froide ou même à 50°C. On peut donc laver des centaines de colonies pour ne retenir que celles qui forment une masse insoluble.

Il est ainsi possible d'isoler des souches microbiennes bonnes productrices et de les améliorer par transfert génétique.

Les bactéries capables de synthétiser des polygalactanes proches de l'agarose à partir des oligosaccharides de cet agarose peuvent aussi faire cette synthése à partir d'oligosaccharides provenant d'autres matières premières.

Il est donc avantageux de choisir des matières premières contenant déjà des D et L galactose. Les produits naturels bon marché contenant ces hexoses sont nombreux: par exemple des algues non productrices d'agar, comme les fucus qui contiennent des fucosanes (séquences de 6-désoxy-D-galactose et de 6-désoxy-L-galactose ou D et L-fucose), ainsi que les algues productrices d'agar-agar, on sépare alors l'agar-agar formé selon ces procédés habituels et l'on utilise le reste (habituellement mis au rebut) pour faire du PGS-O.

La transformation du 6-désoxy-D-galactose et du 6-désoxy-L-galactose en D-galactose et en 3,6-anhydro-L-galactose est obtenue par voie chimique et biologique, selon un procédé connu.

Outre les algues, le nombre d'organismes terrestres (végétaux et microorganismes) produisant des séquences de D et L-galactose ou dérivé du galactose (pectines) est considérable. Le choix de la matière première dépend donc de la région géographique ou de la nature de l'industrie.

Seules les séquences ou alternent des L et des D galactoses sont à retenir. Outre les algues productrices d'agar-agar, on peut utiliser, par exemple, des algues non productrices d'agar-agar (fucus qui pousse où il n'y a pas d'algues donnant de l'agar-agar) et modifier, chinquernent, les séquences de façon à faire alterner les L et les D galactoses.

Le procédé de fabrication industrielle du polygalactane de synthèse-oxyde va maintenant être décrit en détail, en référence à la figure 2 du dessin schématique annexé, sur laquelle (2) répresente le récipient de préparation, (3) une membrane poreuse, (4) un lit d'oxydes métalliques, (5) le milieu nutritif contenant les oligosaccharides ou précurseurs et (6) le polygalactane de synthèse-oxyde obtenu.

On prépare une solution stérile des oligosaccharides par exemple une suspension à 10 % du résidu d'une algue productrice d'agar-agar, dont l'agar-agar a déjà été extrait et on ajoute les facteurs de croissance bactériens nécessaires (source d'azote, vitamines, hydrolysat de caséine à 0,2 %...). Dans cette solution (5) on fait cultiver à travers la membrane poreuse, selon les procédés connus de culture sur membrane, les bactéries capables de produire du polygalactane. Sur cette membrane (3) sont étalées des poudres fines d'oxyde d'alumine ($Al_2O_3$ neutre pour chromatographie) et de titane ($TiO_2$) sur lesquelles sont fixés les initiateurs. L'ensemble forme un lit (4) oû les bactéries cultivent en adhérant aux grains d'oxydes. Les chaînes d'oligosaccharides s'allongent progressivement et forment une nappe continue (6) renfermant oxydes et bactéries.

Périodiquement, la nappe (6) est récoltée, autoclavée pour tuer les bactéries et détruire les activités enzymatiques. Le polygalactane formé étant insoluble dans l'eau froide, le produit est lavé. Seule la partie insoluble est conservée, elle est essorée et donne un produit pâteux. Cette pâte est traitée à l'alcool méthylique et elle est séchée à haute température (140°C) dans des conditons de pH très strictes pour favoriser les formations de liaisons hydrogène sur des films de polytétrafluoroéthylène. Sur ces films, en séchant, les polygalactanes forment une pellicule qui ne doit pas dépasser un centième de millimètre d'épaisseur.

Selon la température de culture, le pH, la source d'azote, l'origine des polysaccharides, le temps de croissance.... les chaînes sont plus ou moins longues. Liées aux oxydes, les chaînes forment une trame dont la dureté augmente avec leur longueur et leur degré de liaison.

Lorsque les chaînes sont très longues, on peut eliminer les oxydes et obtenir des gels transparents.

La définition de tous les paramètres permet de définir des polygalactanes très reproductibles.

La présence de grains d'oxydes auxquels les chaînes de PGS sont liées confert au gel une porosité plus grande que celle de l'agar-agar naturel, mais la trame reste imperméable aux bactéries. Seules les substances chimiques utilisées dans les milieux de culture y diffusent plus vite, ce qui constitue un avantage pour la préparation de ces milieux.

On peut par ailleurs estimer que plus les chaénes de PGS-O sont courtes:
- plus elles sont faciles à obtenir, donc peu onéreuses;
- plus le point de fusion est bas;
- plus le gel est fragile;
- plus il faut de PGS-O matière sèche pour obtenir un gel solide;
- plus la proportion des oxydes doit être grande pour avoir un gel solide;
- plus la porosité est grande.

Inversement, plus les chaînes de PGS-O sont grandes:
- plus elles sont difficiles à obtenir, donc plus elles sont onéreuses;
- plus les quantités d'oxydes peuvent être réduites, ce qui a pour avantage d'éclaircir le gel et de le rendre presque transparent.

En théorie, les gels composés de longues chaînes de polygalactane et rendus transparents peuvent avoir les mêmes qualités que l'agarose (mais ceci au détriment du prix de revient).

La quantité de PGS-O par litre, la proportion des oxydes dans le PGS-O, la longueur estimée des chaînes de

polygalactane, permettent d'obtenir toute une gamme de qualités convenant à des applications différentes et qu'il est possible de prédéterminer selon le tableau ci-après, dans lequel W est le poids de PGS et x,y et z les poids respectifs des différents oxydes (par exemple, x = poids en $Al_2O_3$, y = poids en $TiO_2$ et z = poids en $SiO_2$).

Qualités du PGS-O

| | | | | | |
|---|---|---|---|---|---|
| W | 60 | 60 | 84 | 66 | 30 |
| x | 20 | 18 | 2 | 17 | 30 |
| y | 20 | 20 | 10 | 16 | 30 |
| z | 0 | 2 | 4 | 1 | 10 |

L'application de la substance gélifiante selon l'invention à la réalisation de milieux de culture va maintenant être décrite à l'aide des exemples suivants, qui l'illustrent sans la limiter.

**Exemple 1.** Préparation d'une boîte de Pétri contenant un milieu de MAC CONKEY.

Formule pour cent:

- PGS-O 66.17.16.01 33,62
c'est-à-dire:
66 g de PGS
17 g de $Al_2O_3$
16 g de $TiO_2$
1 g de $SiO_2$
- Peptone 36,34
- Sels biliaires 2,72
- Chlorure de sodium 9,08
- Lactose 18,17
- Rouge neutre 0,052
- Cristal violet 0,018

. Verser une dose stérile de ce milieu, c'est-à-dire 1,376 g dans 25 ml d'eau distillée stérile.
. Porter à 80° C pendant 1 minute.
. Couler dans une boîte de Pétri de 9 cm de diamètre.
. Laisser se gélifier; le milieu est alors prêt à être utilisé.

Résultats.
- Milieu opaque rose saumon clair.
- Les colonies lactose + sont rouges.
- Les colonies lactoses - sont jaunes.
- Seules les entérobactéries se développent.

Par rapport à un milieu de MAC CONKEY en agar-agar naturel, les résultats sont les mêmes mais les colonies sont plus visibles sur le fond opaque quelles que soient leurs tailles.

**Exemple 2**. Préparation de milieu à double couche.

On peut préparer des milieux constitués de deux gels PGS-O de qualités différentes, superposées.

1er cas.
On coule une première couche épaisse, poreuse, absorbante et peu onéreuse qui sert de réserve d'eau et d'éléments nutritifs avec PGS-O 30.30.30.10, c'est-à-dire:

Couche A
30 % de PGS
30 % de $Al_2O_3$
30 % de $TiO_2$
10 % de $CaHPO_4$

On coule par dessus une couche très fine de 1 à 2 mm d'un PGS-O très dense, translucide mais onéreux pour

obtenir de belles colonies: PGS-O 96.02.02, c'est-à-dire:

Couche B
96 % de PGS
2 % de $Al_2O_3$
2 % de $TiO_2$

On peut dailleurs remplacer la couche supérieure translucide par une couche d'agarose très pure.

L'ensemble réalise une économie par rapport à un milieu constitué uniquement d'agar-agar (application pour la culture de plantules où des kilogrammes de milieux sont nécessaires).

2ème cas.

On coule une première couche donnant un gel ferme et nutritif (couche A) qui est fondue à 100° C.

- Après gélification, on coule une deuxième couche d'un gel à point de fusion le plus bas possible, par exemple 60° C (couche B) et contenant des globules rouges.

| | Couche A | Couche B |
|---|---|---|
| PGS | 66 | 40 - fibres courtes |
| $Al_2O_3$ | 17 | 30 |
| $TiO_2$ | 16 | 30 |
| $SiO_2$ | 1 | - |

On réalise ainsi une économie sur le produit le plus cher: les globules rouges et une facilité de réalisation parce que les globules rouges sont très sensibles à la chaleur.

**Exemple 3**

Les PGS-O contenant les chaînes de PGS très courtes et certaines proportions d'oxydes métalliques, forment des gels où les substances chimiques diffusent plus rapidement que dans l'agar-agar. Ces gels sont suffisamment solides pour permettre la culture en surface des bactéries ou des microorganismes. On peut réaliser avec eux des milieux de cultures en continu:

- soit en coulant le gel sur un support contenant ces milieux nutritifs, par exemple un papier buvard imprégné de substances desséchées. La dissolution et la diffusion des substances se fait très rapidement;

- soit en coulant le gel sur un support poreux comme un papier buvard ou un support poreux récupérable (par exemple en acier inox fritté) imprégné ensuite de substances nutritives, ce qui permet, si on renouvelle cette imprégnation, de réaliser des cultures continues de colonies de microorganismes et en particulier de plantules. Ce procédé est très avantageux pour la recherche de mutants bactériens ou végétaux.

Les avantages de la nouvelle substance gélifiante selon l'invention ressortent bien de ce qui précède; on peut les résumer ainsi:

Le PGS-O, qui est soluble comme les poudres nutritives sans être autoclavé, permet de conditionner, dans un même récipient stérile et manipulable stérilement, une quantité désirée de milieux de culture (poudre stérile de substances nutritives).

Puisqu'il est fabriqué par synthèse, ses caractéristiques physicochimiques sont plus faciles à maîtriser; il permet donc la fabrication de milieux de culture mieux définis et mieux normalisés.

A l'état sec, il absorbe les substances nutritives du milieu à sa surface (en raison de la possibilité de le présenter sous forme de petites paillettes pulvérulentes). Ceci permet de le conditionner directement mélangé avec les substances nutritives des milieux de culture, soit sous forme d'un mélange de poudres stériles dans un sachet, soit sous forme de granulés ou de comprimés, soit sous forme d'un mélange de poudres, de granules et de comprimes dans un même sachet.

Le microbiologiste peut ainsi stocker à la température ambiante et pendant un temps très long (supérieur à un an) une très grande variété de milieux de culture desséchés, qu'il peut préparer en quelques minutes et sans aucune perte en fonction de ses besoins.

**Revendications** pour les états contractants (BE, CH, DE, FR, GB, IT, LI, LU, NL, SE).

1. Substance gélifiante, caractérisée en ce qu'elle est essentiellement constituée de chaînes enchevétrees et relativement courtes dont le nombre de disaccharides varie entre 19 et 50, de polygalactanes non ramifiées, où alternent les formes D et L, réunissant des grains d'oxydes métalliques, oxydes et polygalactanes ainsi réunis formant un complexe et non un simple mélange et en ce que ladite substance est insoluble dans l'eau à 25° C et

soluble dans l'eau à 80° C.

2. Substance gélifiante selon la revendication 1, caractérisée en ce que les oxydes métalliques sont choisis parmi les oxydes d'aluminium, de silicium et de titane.

3. Substance gélifiante selon les revendications 1 et 2, caractérisée en ce que la proportion d'oxydes métalliques par rapport aux polygalactanes est inversement proportionnelle à la longueur des chaînes des polygalactanes ou à leur poids moléculaire et se situe entre 10 et 80 %.

4. Substance gélifiante selon l'une quelconque des revendications 1 à 3, caractérisée en ce qu'elle est compatible avec la granulation et la compression et favorise ces deux procédés.

5. Substance gélifiante selon les revendications 1 à 4, caractérisée en ce qu'à l'état sec elle forme des films de moins de un centième de millimètre d'épaisseur.

6. Substance gélifiante selon l'une quelconque des revendications 1 à 5, caractérisée en ce qu'elle donne des solutions dans l'eau dès 80° C.

7. Procédé de préparation de la substance gélifiante selon l'une quelconque des revendications 1 à 6, caractérisé en ce qu'on utilise des microorganismes producteurs d'enzymes et notamment d'hexose transférase pour synthétiser, à partir d'oligosaccharides provenant de matières premières contenant déjà des D et L galactoses, un squelette de polygalactane non ramifié où alternent les formes D et L, la culture desdits microorganismes étant effectuée à travers une membrane poreuse sur laquelle sont étalées des poudres fines d'oxydes métalliques, une étape de sélection étant ensuite effectuée pour ne retenir que les colonies insolubles à l'eau jusqu'à 50° C.

8. Procédé selon la revendication 7, caractérisé en ce que l'on prépare stérilement un milieu de culture avec les oligosaccharides provenant de l'hydrolyse de l'agarose, en ce que l'on isole à la surface de ce milieu des variétés myxoïdes de bactéries connues pour produire des polysaccharides capsulaires, et en ce que l'on recherche par screening et par clonage les souches capables à partir des oligosaccharides du milieu, de faire des chaînes plus longues.

9. Application de la substance gélifiante selon l'une quelconque des revendications 1 à 6 à la realisation de milieux de culture.

10. Application selon la revendication 9, caractérisée en ce que les milieux de culture sont préparés sous forme de doses unitaires.

**Revendications** pour l'état contractant AT

1. Procédé de préparation d'une substance gélifiante, caractérisé en ce qu'on utilise des microorganismes producteurs d'enzymes et notamment d'hexose transférase pour synthétiser, à partir d'oligosaccharides provenant de matières premières contenant déjà des D et L galactoses, un squelette de polygalactane non ramifié où alternent les formes D et L, la culture desdits microorganismes étant effectuée à travers une membrane poreuse sur laquelle sont étalées des poudres fines d'oxydes métalliques, une étape de sélection étant ensuite effectuée pour ne retenir que les colonies insolubles à l'eau jusqu,à 50° C.

2. Procédé selon la revendication 1, caractérisé en ce que l'on prépare stérilement un milieu de culture avec les oligosaccharides provenant de l'hydrolyse de l'agarose, en ce que l'on isole à la surface de ce milieu des variétés myxoïdes de bactéries connues pour produire des polysaccharides capsulaires et en ce que l'on recherche par screening et par clonage les souches capables à partir des oligosaccharides du milieu, de faire des chaînes plus longues.

3. Procédé selon la revendication 1 et la revendication 2, caractérisé en ce que les oxydes métalliques sont choisis parmi les oxydes d'aluminium, de silicium et de titane.

4. Procédé selon l'une quelconque des revendications 1 à 3, caractérisé en ce que la proportion d'oxydes métalliques par rapport aux polygalactanes est inversement proportionnelle à la longueur des chaînes des polygalactanes ou à leur poids moléculaire et se situe entre 10 et 80 %.

5. Application de la substance gélifiante obtenue par mise en oeuvre du procédé selon l'une quelconque des revendications 1 à 4 à la réalisation de milieux de culture.

6. Application selon la revendication 5, caractérisée en ce que les milieux de culture sont préparés sous forme de doses unitaires.

**Patentansprüche** für die Vertagsstaaten BE, CH, DE, FR, GR, IT, LI, LU, NL, SE.

1. Gelierende Substanz, dadurch gekennzeichnet, daß sie im wesentlichen gebildet wird aus verfilzten und relativ kurzen Ketten, bei denen die Anzahl der Disaccharide zwischen 19 und 50 beträgt, von nicht verzweigten Polygalactanen mit alternierenden D- und L-Formen, die durch Metalloxidkörner verbunden sind, wobei so verbundene Oxide und Polygalactane einen Komplex und nicht ein einfaches Gemisch bilden und daß die Substanz in Wasser von 25° C unlöslich und in Wasser von 80° C löslich ist.

2. Gelierende Substanz nach Anspruch 1, dadurch gekennzeichnet , daß die Metalloxide ausgewählt sind aus den Oxiden von Aluminium, Silizium und Titan.

3. Gelierende Substanz nach einem der Ansprüche 1 und 2, dadurch gekennzeichnet, daß der Anteil der Metalloxide im Verhältnis zu den Polygalactanen umgekehrt proportional zu der Kettenlänge der Polygalactane oder ihrem Molekulargewicht ist und zwischen 10 und 80 % beträgt.

4. Gelierende Substanz nach einem der Ansprüche 1 bis 3, dadurch gekennzeichnet, daß sie Granulation und Kompression verträgt und daß diese beiden Verfahren bevorzugt sind.

5. Gelierende Substanz nach einem der Ansprüche 1 bis 4, dadurch gekennzeichnet, daß sie im trockenen Zustand Filme von weniger als 1/100 mm Stärke ausbildet.

6. Gelierende Substanz nach einem der Ansprüche 1 bis 5, dadurch gekennzeichnet, daß sie in Wasser von 80° C Lösungen ergibt.

7. Verfahren zur Herstellung einer gelierenden Substanz nach einem der Ansprüche 1 bis 6, dadurch gekennzeichnet, daß man Mikroorganismen verwendet, die Enzyme und insbesonders Hexosetransferase produzieren, um von den Oligopolysacchariden, die aus den Ausgangsstoffen stammen, welche schon D- und L-Galactosen enthalten, ein unverzweigtes Polygalactanskelett zu synthetisieren, bei dem D- und L-Formen alternieren, wobei die Mikroorganismenkultur durch eine poröse Membran hindurch verwendet wird, auf der feine Puder von Metalloxiden verteilt sind, und daß man eine Selektionsstufe danach durchführt, um nur die Kolonien zurückzuhalten, die in Wasser bis 50° C unlöslich sind.

8. Verfahren nach Anspruch 7, dadurch gekennzeichnet , daß man ein steriles Kulturmilieu mit den Oligosacchariden herstellt, die aus der Hydrolyse von Agarose stammen und daß man auf der Oberfläche des Milieus verschiedene Myxoide von Bakterien isoliert, von denen bekannt ist, daß sie kapsuläre Polysaccharide herstellen und daß man durch Screenen und Klonieren die Stämme findet, die ausgehend von Oligosacchariden des Milieus geeignet sind, längere Ketten auszubilden.

9. Anwendung der gelierenden Substanz nach einem der Ansprüche 1 bis 6 zum Herstellen eines Kulturmediums.

10. Anwendung nach Anspruch 9, dadurch gekennzeichnet , daß die Kulturmedien in Form von einheitlichen Dosen hergestellt werden.

**Patentansprüche** für den Vertragsstaat AT

1. Verfahren zur Herstellung einer gelierenden Substanz, dadurch gekennzeichnet , daß man Mikroorganismen verwendet, die Enzyme und insbesonders Hexosetransferase produzieren, um von den Oligopolysacchariden, die aus den Ausgangsstoffen stammen, welche schon D- und L-Galactosen enthalten, ein unverzweigtes Polygalactanskelett zu synthetisieren, bei dem D- und L-Formen alternieren, wobei die Mikroorganismenkultur durch eine poröse Membran hindurch verwendet wird, auf der feine Puder von Metalloxiden verteilt sind, und daß man eine Selektionsstufe danach durchführt, um nur die Kolonien zurückzuhalten, die in Wasser bis 50° C unlöslich sind.

2. Verfahren nach Anspruch 1, dadurch gekennzeichnet , daß man ein steriles Kulturmilieu mit den Oligosacchariden herstellt, die aus der Hydrolyse von Agarose stammen und daß man auf der Oberfläche des Milieus verschiedene Myxoide von Bakterien isoliert, von denen bekannt ist, daß sie kapsuläre Polysaccharide herstellen und daß man durch Screenen und Klonieren die Stämme findet, die ausgehend von Oligosacchariden des Milieus geeignet sind, längere Ketten auszubilden.

3. Verfahren nach Anspruch 1 und 2, dadurch gekennzeichnet, daß die Metalloxide ausgewählt sind aus den Oxiden von Aluminium, Silizium und Titan.

4. Verfahren nach einem der Ansprüche 1 bis 3, dadurch gekennzeichnet, daß der Anteil der Metalloxide im Verhältnis zu den Polygalactanen umgekehrt proportional der Kettenlänge der Polygalactane oder ihres Molekulargewichtes ist und 10 bis 80 % beträgt.

5. Anwendung der gelierenden Substanz, erhalten nach dem Verfahren nach einem der Ansprüche 1 bis 4 zur Herstellung von einem Kulturmilieu.

6. Anwendung nach Anspruch 5, dadurch gekennzeichnet , daß das Kulturmilieu in Form von gleichen Dosen hergestellt wwird.

**Claims** for the Contracting States BE, CH, DE, FR, GB, IT, LI, LU, NL, SE.

1. A gelling substance, characterized in that it consists essentially of relatively short entangled chains, the number of disaccharides of which varies from 19 to 50, and of non-ramified polygalactanes in which the dextro- and laevo-rotary forms alternate, joining together grains of metallic oxides, the oxides and polygalactanes thus joined forming a complex and not a simple mixture, and in that the said substance is insoluble in water at 25° C and soluble in water at 80° C.

2. A gelling substance according to Claim 1, characterized in that the metallic oxides are selected from the oxides of aluminium, silicon and titanium.

3. A gelling substance according to Claims 1 and 2, characterized in that the proportion of metallic oxides relatively to the polygalactanes is inversely proportional to the length of the polygalactane chains or to their

molecular weights and varies bertween 10 and 80 %.

4. A gelling substance according to any of Claims 1 to 3, characterized in that it is compatible with granulation and compression and favours these two processes.

5. A gelling substance according to Claims 1 to 4, characterized in that in the dry state it forms films less than one-hundredth of a millimetre thick.

6. A gelling substance according to any of Claims 1 to 5, characterized in that it produces agueous solutions at temperatures above 80° C.

7. A process for the preparation of the gelling substance according to any of Claims 1 to 6, characterized in that use is made of microorganisms that produce enzymes, and particularly produce hexose transferase to synthesize, starting from oligosaccharides providing the raw material that already contains dextro- and laevo-rotary galactoses, a non-ramified skeleton of polygalactane in which the dextro- and laevo-rotary forms alternate, the culture of the said microorganisms being performed through a porous membrane on which finely-powdered metallic oxides are scattered, a selection process being subsequently performed to retain only those colonies that are insoluble in water up to 50° C.

8. A process according to Claim 7, characterized in that a culture medium is prepared under sterile conditions from oligosaccharides obtained by the hydrolysis of agarose, by isolating on the surface of this medium myxoid varieties of bacteria that are known to produce capsular polysaccharides, and by screening and cloning selecting those colonies of microbes that are capable of making longer chains from the oligosaccharides of the medium.

9. The use of the gelling substance according to any of Claims 1 to 6 to produce culture media.

10. The use according to Claim 9, characterized in that the culture media are prepared in the form of unit doses.

**Claims** for the Contracting State AT

1. A process for the preparation of a gelling substance characterized in that use is made of microorganisms that produce enzymes, and particularly produce hexose transferase to synthesize, starting from oligosaccharides providing the raw material that already contains dextro- and laevo-rotary galactoses, a non-ramified skeleton of polygalactane in which the dextro- and laevo-rotary forms alternate, the culture of the said microorganisms being performed through a porous membrane on which finely-powdered metallic oxides are scattered, a selection process being subsequently performed to retain only those colonies that are insoluble in water up to 50° C.

2. A process according to Claim 1, characterized in that a culture medium is prepared under sterile conditions from oligosaccharides obtained by the hydrolysis of agarose, by isolating on the surface of this medium myxoid varieties of bacteria that are known to produce capsular polysaccharides, and by screening and cloning selecting those colonies of microbes that are capable of making longer chains from the oligosaccharides of the medium.

3. A process according to Claim 1 and Claim 2, characterized in that the metallic oxides are selected from the oxides of aluminium, silicon and titanium.

4. A process according to any of Claims 1 to 3, characterized in that the porportion of metallic oxides relatively to the polygalactanes is inversely proportional to the length of the polygalactane chains or to their molecular weights and varies between 10 and 80 %.

5. The use of the gelling substance obtained by making use of the procedure according to any of Claims 1 to 4 to produce culture media.

6. The use according to Claim 5, characterized in that the culture media are prepared in the form of unit doses.

FIG.1

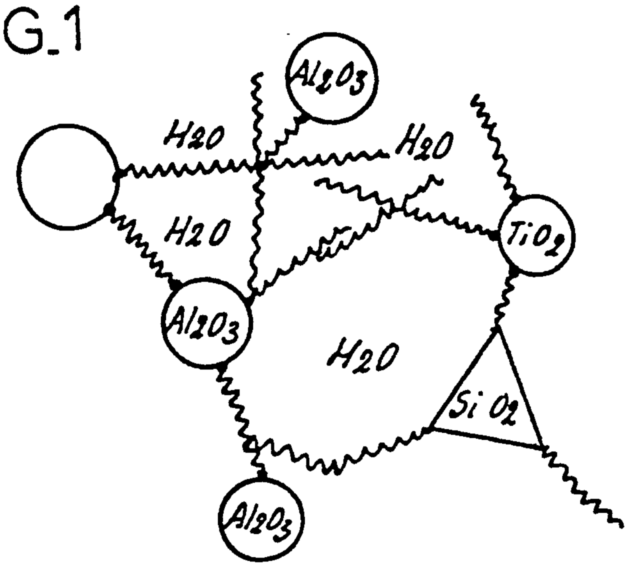
Al2O3
H2O
H2O
H2O
TiO2
Al2O3
H2O
Si O2
Al2O3

FIG.2

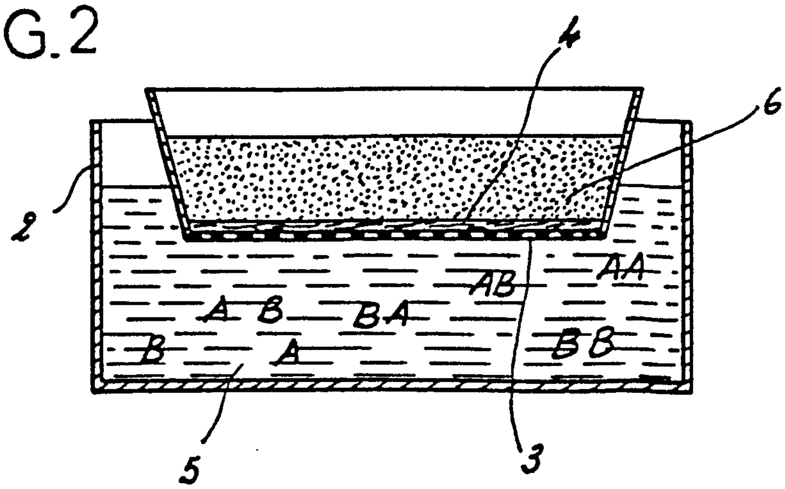
4
6
2
AB
AA
A B
BA
B
A
BB
5
3

FIG_3

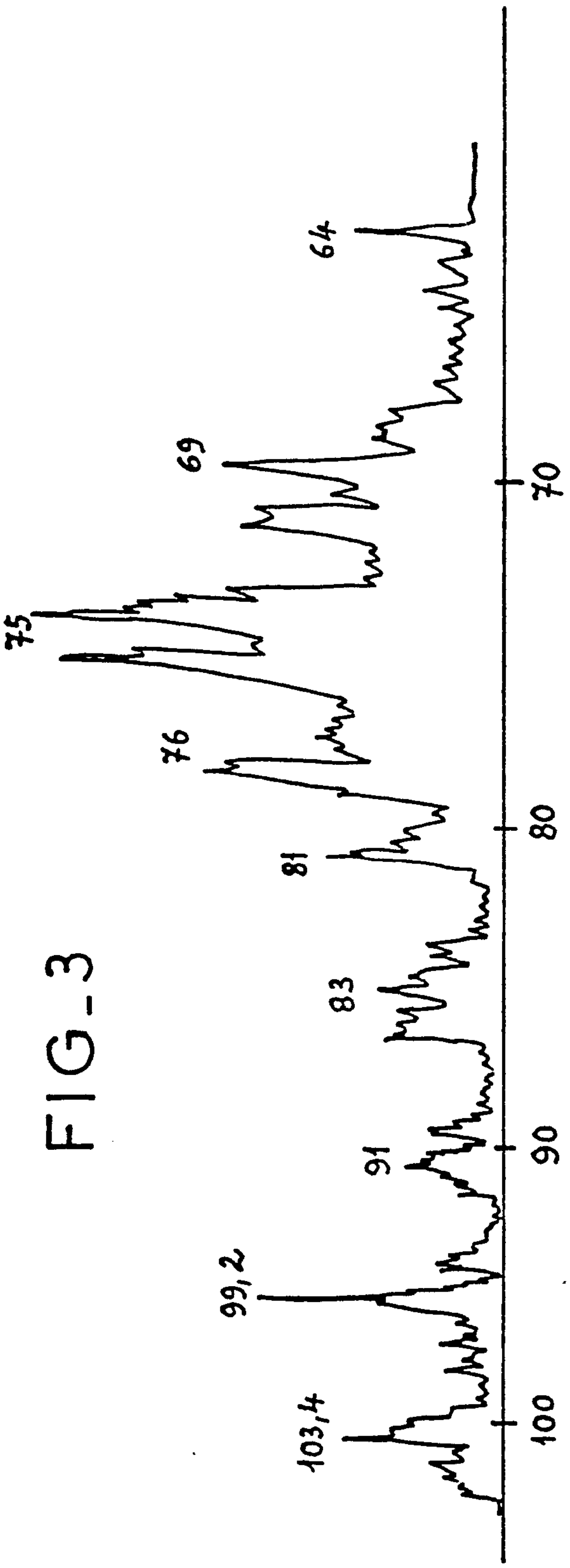
75
76
69
64
81
83
91
99,2
103,4
70
80
90
100

FIG_4

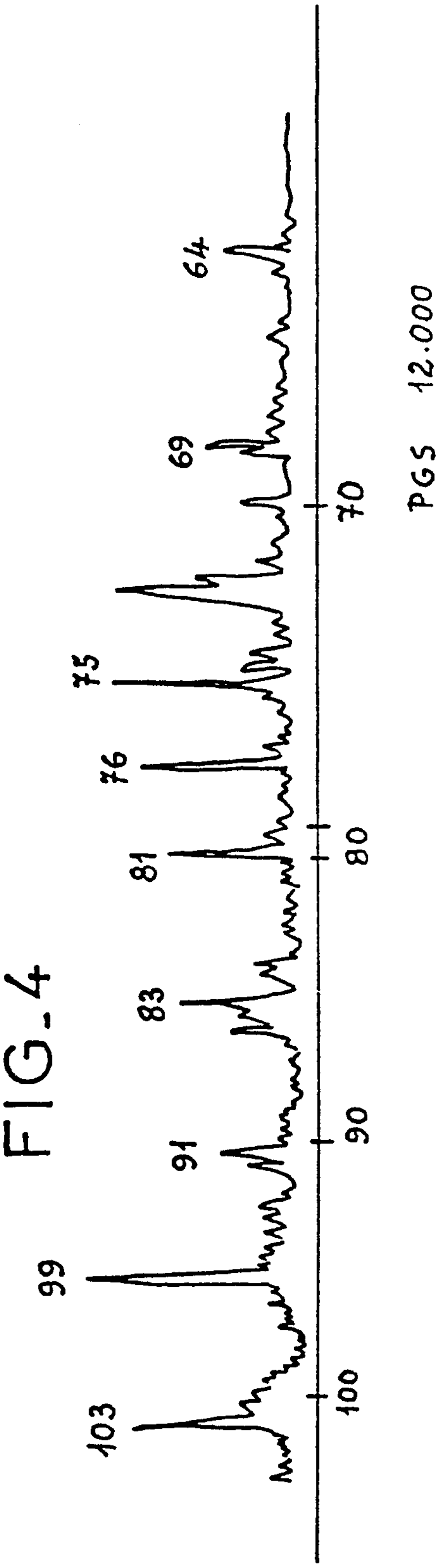
75
76
69
64
81
83
91
99
103
70
80
90
100
PGS 12.000